# EUROPEAN PATENT SPECIFICATION

(11) **EP 1 618 890 B1**
(45) Date of publication and mention of the grant of the patent: **06.10.2010**
(21) Application number: 04450149.2
(22) Date of filing: 20.07.2004
(51) Int. Cl.: A61K 39/395, A61K 39/39, A61P 35/00, C07K 16/30

(54) **Use of antibodies in a very low dose for the vaccination against cancer**
Verwendung von Antikörpern in einer sehr geringen Dosis zur Impfung gegen Krebs
Utilisation d'anticorps en très faibles doses pour vaccination contre le cancer

(43) Date of publication of application: 25.01.2006
(73) Proprietor: ALTROPUS GMBH, 4144 Arlesheim (CH)
(72) Inventor: Loibner, Hans, 1238 Vienna (AT); Himmler, Gottfried, 1180 Vienna (AT); Schuster, Manfred, 2191 Schrick (AT)
(74) Representative: Sonn & Partner Patentanwälte

(56) References cited:
- EP-A- 1 140 168
- EP-A- 1 230 932
- WO-A-00/41722

## Description

The present invention relates to the use of antibodies directed against human cellular membrane antigens at very low dosage for the preparation of a pharmaceutical composition for the vaccination against cancer. The invention further relates to a vaccine containing the antibodies at the very low dosage.

Monoclonal antibodies (mAb) have been widely used for immunotherapy of a variety of diseases, among them infectious and autoimmune disease, as well as conditions associated with tumours or cancer. Using hybridoma technology, mAb directed against a series of antigens have been produced in a standardized manner. A multitude of tumor-associated antigens (TAAs) are considered suitable targets for mAb and their use for the diagnosis of cancer and therapeutic applications. TAAs are structures that are predominantly expressed on the cell membrane of tumor cells and thus allow differentiation from non-malignant tissue.

In the course of the discovery and the subsequent characterization of the most varied TAA, it has turned out that they have important functions as regards cancer cells. They enable the degenerate cells to show properties characteristic of the malignant phenotype, such as an increased capability for adhesion, which play an important role in establishing metastases. However, such antigens can, at certain stages, also be expressed on normal cells where they are responsible for the normal functions of these cells. Without laying claim to completeness, some examples of such antigens are listed in the following:
- N-CAM (Neuronal Cell Adhesion Molecule), which is often expressed on tumors of neuronal origin and which effects homophilic adhesion (J. Cell Biol. 118 (1992), 937).
- The Lewis Y carbohydrate antigen, which occurs on the majority of tumors of epithelial origin, but which also plays an important role during the fetal development of epithelial tissues. It has been shown that the expression of this antigen in lung cancer is strongly associated with an unfavorable prognosis since Lewis Y positive cancer cells obviously have a higher metastatic potential (N. Engl. J. Med. 327 (1992), 14).
- CEA (Carcino Embryonic Antigen), which often occurs on epithelial tumors of the gastrointestinal tract and which has been identified as a self-adhesion molecule (Cell 57 (1989), 327).
- Ep-CAM (Epithelial Cell Adhesion Molecule), which is expressed on nearly all tumors of epithelial origin, but which also occurs on a large number of normal epithels. It has been characterized as a self-adhesion molecule and can therefore be classified as a pan-epithelial adhesion antigen (J. Cell Biol. 125 (1994), 437).

Tumor-associated antigens are structures which are predominantly presented by tumor cells and thereby allow a differentiation from non-malignant tissue. Preferably, such a tumor-associated antigen is located on or in the cell membrane of a tumor cell. This does, however, not exclude the possibility that such antigens also occur on non-degenerate cells. The tumor-associated antigens can, for example, be polypeptides, in particular glycosylated proteins, or glycosylation patterns of polypeptides. Other structures which may represent a tumor-associated antigen are, e.g., glycolipids. These include, for example, gangliosides, such as GM2. Moreover, tumor-associated antigens may be represented by changes in the composition of lipids of the cell membrane which may be characteristic of cancer cells.

Further examples of tumor-associated antigens are Sialyl Tn carbohydrate, Globo H carbohydrate, gangliosides such as GD2/GD3/GM2, Prostate Specific Antigen (PSA), CA 125, CA 19-9, CA 15-3, TAG-72, EGF receptor, Her2/Neu receptor, p97, CD20 and CD21. Monoclonal antibodies directed against all these antigens are available. Further tumor-associated antigens are described, e.g., in DeVita et al. (Eds., "Biological Therapy of Cancer", 2. Edition, Chapter 3: Biology of Tumor Antigens, Lippincott Company, ISBN 0-397-51416-6 (1995)).

Whether human TAAs detected by xenogeneic mAbs are capable of inducing an antitumor immune response in cancer patients, and whether such antigens are indeed related to the response to autologous tumors in cancer patients, depends on the nature of the respective TAA and is still not fully understood. TAAs which are either naturally immunogenic in the syngeneic host or can be made immunogenic might potentially be used to induce antitumor immunity for therapeutic and possibly prophylactic benefit.

For passive immunotherapy mAbs are administered systemically to a patient in a suitable amount to directly bind to a target. Thus an immune complex is formed and through a series of immune reactions the cell or organism afflicted with the target is killed. The therapeutic effect is depending on the concentration of the mAbs in the circulation and the biological half-life, which is usually quite short. It is therefore necessary to repeat the administration within an appropriate timeframe. If xenogeneic mAbs, such as murine antibodies are used, adverse reactions are however expected, possibly leading to anaphylactic shock. Therefore, such immunotherapies are employed for a limited time only.

Active immunization regimens activate the immune system of patients in a specific way. Following the administration of an antigen that resembles a specific target the patients humoral and T-cell specific immune response induces defense mechanisms to combat the target in vivo. Vaccine antigens of various types and against a wide variety of different diseases are well known in the art. For example vaccination against Hepatitis B using vaccines containing surface hepatitis B antigens are well known. It has been shown that high dose ranges of antigens used for vaccination as well as low-dose vaccination can give sufficient rates of seroconversion (Parish D.C. et al., 1991, Southern Medical Journal, 84, 426-430; Goudeau A. et al., 1984, The Lancet, 10, 1091-1092).

There are also Mannan-Mucin fusion proteins described, which are used for generating cytotoxic T cells. It was shown that depending on the dosage of the adiministered fusion protein to mice, either almost only cellular immunity (low doses) or only humoral immunity (high doses) was induced (Pietersz G.A. et al., 1998, Caner Immunol. Immunother., 45, 321-326)

For active immunization these antigens are usually presented in an immunogenic formulation to provide a vaccine. Antigens mimicking the targets have either similarities in the primary and secondary sequence of the targets or fragments thereof. Mimotopes or mimotopic antigens, however, have similarities in the tertiary structure of the target.

Exemplary mimotopes are anti-idiotypic antibodies or mimotopic antibodies that imitate the structure of an antigen, which is considered as target for the immune system. Idiotypic interactions strongly influence the immune system. The unique antigenic determinants in and around the antigen-combining site of an immunoglobulin (Ig) molecule, which make one antibody distinct from another, are defined as idiotopes. All idiotopes present on the variable portion of an antibody are referred to as its idiotype (id). The molecular structure of an idiotype has been localized to both the complementary determining regions and the framework regions of the variable domain and is generally but not always contributed to by both the heavy and the light chains of an immunoglobulin in specific association.

Idiotypes are serologically defined entities. Injection of an antibody (Ab1) into a syngeneic, allogeneic, or xenogeneic recipient induces the production of anti-idiotypic antibodies (Ab2). With regard to idiotype/anti-idiotype interactions a receptor-based regulation of the immune system was postulated by Niels Jerne (Ann. Immunol. 125C, 373, 1974). His network theory considers the immune system as a collection of 1g molecules and receptors on T-lymphocytes, each capable of recognizing an antigenic determinant (epitope) through its combining site (para-tope), and each capable of being recognized by other antibodies or cell-surface receptors of the system through the idiotopes that it displays.

Many studies have indeed demonstrated that idiotypic and anti-idiotypic receptors are present on the surface of both B- and T-lymphocytes as well as on secreted antibodies. An overview about anti-idiotypic antibodies used for the development of cancer vaccines is presented by Herlyn et al. (in vivo 5: 615-624 (1991)). The anti-idiotypic cancer vaccines contain either monoclonal or polyclonal Ab2 to induce anti-tumor immunity with a specificity of selected TAA.

When the binding between Ab1 and Ab2 is inhibited by the antigen to which Ab1 is directed, the idiotype is considered to be binding-site-related, since it involves a site on the antibody variable domain that is engaged in antigen recognition. Those idiotypes which conformationally mimic an antigenic epitope are called the internal image of that epitope. Since both an Ab2 and an antigen bind to the relevant Ab1, they may share a similar three-dimensional conformation that represents the internal image of the respective antigen. Internal image anti-idiotypic antibodies in principle are substitutes for the antigen from which they have been derived via the idiotypic network. Therefore these surrogate antigens may be used in active immunization protocols. The anti-idiotypic antibodies offer advantages if the original antigen is not sufficiently immunogenic to induce a significant immune response. Appropriate internal image anti-idiotypic antibodies that mimic a non-immunogenic carbohydrate antigen are especially useful for certain vaccination approaches.

Tumor associated antigens are often a part of "self" and evoke a very poor immune response in cancer patients. In contrast, internal image anti-idiotypic antibodies expressing three-dimensional shapes, which resemble structural epitopes of the respective TAA, are recognized as foreign molecules in the tumor-bearing host.

The immune response raised by therapeutic or even prophylactic immunization with appropriate anti-id MABs, thus may cause anti-tumor immunity.

Mimotopic antibodies are alike anti-idiotypic antibodies. They too resemble a target structure and may possibly activate the immune system against the target. The EP-B1-1 140 168 describes mimotopic antibodies against human cellular membrane antigens to produce antitumor immunity in cancer patients. These antibodies are directed against the EpCAM, NCAM or CEA antigens; each of these targets is well known to be tumor associated.

Therapeutic immunization against cancer with MABs may be especially successful in earlier stages of the disease: At the time of surgery of a primary tumor, frequently occult single tumor cells already have disseminated in various organs of the patient. These micrometastatic cells are known to be the cause for the later growth of metastases, often years after diagnosis and surgical removal of all clinically proven tumor tissue. So far in almost all cases metastatic cancer of epithelial origin is incurable.

Therefore an effective treatment of "minimal residual cancer", e.g. destruction of occult disseminated tumor cells or micrometastatic cells in order to prevent the growth of metastases is an urgent medical need. At these stages of the disease (adjuvant setting) conventional chemotherapeutic approaches are rather unsuccessful. However, specific antitumor immunity at the time of minimal residual disease can be obtained by immunization with appropriate MAB. Micrometastatic cells may thus be selectively eliminated by the immune system, leading to an increased relapse-free survival time.

The use of a monoclonal antibody against EpCAM has been described for active immunotherapy of cancer patients (EP 1 140 168), wherein an immunogenic formulation containing the antibody in a dose range of 0.01 to 4 mg is administered.

The technical problem underlying the present invention is to provide means and methods which allow an efficient prophylaxis against or therapy of cancer diseases which are highly economic and highly effective.

This problem has been solved by the provision of the embodiments as characterized in the claims.

Accordingly, the invention relates to the use of antibodies which are directed against EpCAM for the preparation of a pharmaceutical composition for the prophylactic and/or therapeutic vaccination against cancer at a very low dose. This very low dose range of a vaccine formulation containing the antibody means that the antibody concentration is in the range from 0.01 µg to 9 µg, preferably from 0.05 µg to 9 µg, more preferably from 0.05 µg to 5 µg.

In a preferred embodiment, the anti-EpCAM antibody used according to the invention is as described in EP 1 140 168.

Preferably the antibody used according to the invention is a monoclonal antibody. Preferably the formulation according to the invention contains an isolated, monoclonal anti-EpCAM antibody together with a vaccine adjuvant.

As known from the art, the magnitude of immune response using anti-idiotypic antibodies, which is known to be used for vaccination purposes, is highly dose dependent. For example, vaccination with high doses (500 µg) of anti-idiotypic antibodies to an Sendai Virus specific T-cell clone leads to a stronger immune response compared to intermediate doses (5µg). In contrast, low dose immunization (50 ng) resulted in negligible immune response (Ertl H.C., 1989, Viral Immunology, 2, 247-249).

Cancer vaccine formulations containing an antibody directed against human cellular membrane antigens in an immunogenic formulation are known to elicit humoral immune response when administered at a dose of 500µg of antibody per vaccination.

It has now been surprisingly found that a vaccine formulation containing an antibody directed against cellular membrane antigens at a dosage from 0.01 µg/dose to 9 µg/dose can still raise a strong humoral immune response.

Using different very low dosage amounts (5 µg, 0.5 µg, 0.05µg) the induction of a strong overall immune response was shown. Kinetics of the immune response can be differing depending on the dose, but seroconversion can be reached in more than 70%, preferably more than 90%, more preferably more than 99% of the tests.

Seroconversion can be, for example, assayed by differential measurement of the binding of immunoglobulins of a serum from an individual (before and after immunizations) to the antigen used for immunization. If the serum of the individual does in fact show immunoglobulins specific against the antigen that had been applied, seroconversion has proven.

It is known from other vaccine antigens, wherein for example pathogenic structures are isolated and formulated as vaccines, that low dosages can lead to cellular immune response in individuals. When an antibody is used in a vaccine formulation at such a low dosage as it is the case according to the invention, a sufficient immune response, esp. a measureable humoral immune response would not have been expected. Therefore it was highly surprising to the inventors that the antibody according to the invention can still induce a highly sufficient immune response, preferably a humoral immune response.

The term "antibody" relates to antibodies of all possible types, in particular to polyclonal or monoclonal antibodies or also to antibodies produced by chemical, biochemical or gene technological methods. Methods for producing such molecules are known to the person skilled in the art. The way of producing the antibody is not important. Only its binding specificity for a relevant epitope of a cellular membrane antigen is important. Preferably, monoclonal antibodies are used, most preferably monoclonal antibodies of animal origin, in particular of murine origin. It is particularly preferred that the murine monoclonal antibody mAb17-1A is used, which can be produced as described, or an antibody which has the same fine specificity of binding as mAb17-1A. Within the meaning of the present invention, the term "antibody" also includes fragments and derivatives of antibodies wherein these fragments or derivatives recognize a TAA. The therapeutically effective immune response which is induced by the vaccination with suitable antibodies directed against TAA is determined by the binding region of these antibodies, i.e. by their idiotype. Therefore, it is, in principle, also possible to use, instead of intact antibodies, fragments or derivatives of these antibodies for a successful vaccination as long as these derivatives still contain the idiotype of the respective starting-antibody. As examples, without being limiting, can be listed: F(ab)'₂ fragments, F(ab)' fragments, Fv fragments which can be produced either by known biochemical methods (enzymatic cleavage) or by known methods of molecular biology. Further examples are derivatives of antibodies, which can be produced according to known chemical, biochemical or gene technological methods.

Preferably the antibody as used in the formulation according to the invention is produced by hybridoma cells or recombinantly produced (AT599/2003). The antibody used in the formulation according to the invention can also be a multivalent antibody as described in 03/097663. Herein, structures which are tumor associated antigenic structures can be chemically coupled to the antibody. Coupling of the antigenic structures can also be done by glycoengineering methods as known in the art.

These structures can be glycopeptides, carbohydrates, lipids, nucleic acids or ionic groups be covalently coupled. Preferably these structures are selected from the group consisting of SialylTn, Lewis Y, Globo H.

The antibody according to the invention can therefore be an antibody targeting EpCAM (an ab1) or special embodiments of the vaccine according to the invention contain, in particular, anti-idiotypic antibodies as vaccination antigens, i.e. ab2 which are directed against the idiotype of an EpCAM specific antibody (ab1). Anti-idiotypic antibodies provoke in vivo the formation of ab3 antibodies which in turn also target a TAA of a tumor cell, bind thereto and lyse it accordingly.

In this context, the term "derivative", in particular, also includes products which can be produced by chemical linkage of antibodies (antibody fragments) with molecules which can enhance the immune response, such as tetanus toxoid, Pseudomonas exotoxin, derivatives of Lipid A, GM-CSF, IL-2 or by chemical linkage of antibodies (antibody fragments) with lipids for a better incorporation into a liposome formulation. The term "derivative" also includes fusion proteins of antibodies (antibody fragments), which have been produced gene technologically, with polypeptides which can enhance the immune response, such as GM-CSF, IL-2, IL-12, C3d etc. According to the invention, the antibodies can, of course, also be applied in combination with each other. This means that two or more antibodies which recognize different membrane antigens or different epitopes of the same membrane antigen can be administered. The different antibodies can be administered simultaneously (together or separately) or subsequently. Cancer cells often express several TAA at the same time against which suitable antibodies for vaccination are either available or can be generated. In order to obtain an enhanced or possibly synergistic effect of the induced immune response and to minimize the potential danger of the selection of antigen-negative variants and in order to counteract a possible tumor cell heterogenity, it may be advantageous to use a combination of two or more suitable antibodies or their fragments or derivatives simultaneously for vaccination.

In the context of the present invention the term "vaccination" means an active immunization, i.e. an induction of a specific immune response due to administration (e.g. subcutaneous, intradermal, intramuscular, possibly also oral, nasal) of small amounts of an antigen (a substance which is recognized by the vaccinated individual as foreign and therefore immunogenic) in a suitable immunogenic formulation. The antigen is thus used as a "trigger" for the immune system in order to build up a specific immune response against the antigen. In case of the present invention the vaccine is a vaccine formulation containing an antibody directed against a TAA, preferably EpCAM.

In the sense of the present invention a vaccination can, in principle, be either carried out in the therapeutic sense as well as in the prophylactic sense (as is the case with all antimicrobial vaccines). This means that the vaccination against cancer according to the present invention can be understood as both a therapeutic and a prophylactic application. Accordingly, it might optionally be possible to achieve a prophylactic protection against the breakout of a cancer disease by vaccination of individuals who do not suffer from cancer. Individuals to whom such a prophylactic vaccination can be applied are individuals who have an increased risk to develop a cancer disease, although this application is not limited to such individuals. Patients being at risk of cancer can already have developed tumors, either as primary tumors or metastases, or show predisposition for cancer.

As this invention provides the use of a very low dose of the immunogenic formulation, especially the prophylactic vaccination can be an important tool in cancer prophylaxis.

The use according to the present invention differs substantially from the basic possibilities of therapeutic application of antibodies for the treatment of cancer that have been known so far and have been described earlier and allows for an unexpectedly efficient treatment.

The antibody against EpCAM can represent a structural complementary picture of the respective TAA. This means that such an antibody has a structural information of the EpCAM epitope against which it is directed or at least leads to an immune response against structural components of the tumor cell. Thus, if a cancer patient is vaccinated with a suitable immunogenic antibody binding to EpCAM (i.e. for example with a murine MAB against EpCAM), antibodies are produced in the patient which are directed against the antibody used as vaccine. This means that due to such a vaccination, so to say, soluble variants of the epitope of the tumor cell surface structure are generated in the cancer patient, which can be effective as actively induced autologous antibodies for a long period of time and the titer of which can be boosted in suitable intervals by repeated vaccinations.

The formation of new metastases can be suppressed and the dissemination of the disease can, at least, be slowed down thanks to vaccination of cancer patients with suitable antibodies against TAA, preferably EpCAM. In early stages of the disease, for example after a successful surgery of a primary tumor (adjuvant stage), remaining disseminated tumor cells are prevented from establishing themselves as new metastases due to such vaccinations. This allows to prolong the relapse-free survival period and therefore the overall lifetime of such patients. It may optionally be possible to obtain a lifelong protection against the formation of metastases due to such vaccinations and booster vaccinations which are carried out in suitable intervals. Of particular interest are vaccinations of cancer patients with suitable antibodies directed against EpCAM since in these cases, as described above, it is possible to achieve an enhanced therapeutic effect due to an additional direct attack of the induced immune response on the tumor cells.

In a further preferred embodiment, the pharmaceutical composition prepared according to the use of the present invention contains at least one adjuvant commonly used in the formulation of vaccines apart from the antibody. It is possible to enhance the immune response by such adjuvants. As examples of adjuvants, however not being limited to these, the following can be listed: aluminium hydroxide (Alu gel), QS-21, Enhanzyn, derivatives of lipopolysaccharides, Bacillus Calmette Guerin (BCG), liposome preparations, formulations with additional antigens against which the immune system has already produced a strong immune response, such as for example tetanus toxoid, Pseudomonas exotoxin, or constituents of influenza viruses, optionally in a liposome preparation, biological adjuvants such as Granulocyte Macrophage Stimulating Factor (GM-CSF), interleukin 2 (IL-2) or gamma interferon (IFNγ).

Aluminium hydroxide is the most preferred vaccine adjuvant.

The vaccination can be carried out by a single application of the above mentioned dosage. However, preferably the vaccination is carried out by repeated applications. The number of repetitions is in the range from 1 to 12 per year, more preferably in the range from 4 to 8 per year. The dosage can remain the same or can decrease.

Booster vaccinations can be carried out in regular intervals, in principle, lifelong. Suitable intervals are in the range from 6 to 24 months and can be determined by monitoring the titer of the induced antibodies (a booster vaccination should be carried out as soon as the titer of the induced antibodies has dropped significantly).

The administration of the antibody can be carried out according to methods known to the person skilled in the art. Preferably, the pharmaceutical composition containing the antibody is suitable for a subcutaneous, intradermal or intramuscular administration.
Figure 1 shows the immunization antigen specificity measured by mAb17-1A ELISA. P3/03 (5□g mAb17-1A/dose), P4/03 (500ng mAb17-1A /dose), P5/03 (50ng mAb17-1A /dose).
Figure 2 shows the specificity of induced immune response analyzed by mAb17-1A affinity chromatography.
Figure 3 shows immunization antigen dose dependent induced humoral immune responses (IgG) measured by affinity chromatography.

The following examples are describing the invention in more detail, but not limiting the scope of the invention.

### Examples:

### Abbreviations:

- ELISA: Enzyme Linked Immuno Sorption Assay
- HRP: Horse Radish Peroxidase
- IAC: immuno affinity chromatography
- IS: immune serum
- mAb: Monoclonal antibody
- PBS: Phosphate Buffered Saline
- PS: Preserum
- rEpCAM: recombinant epithelial cell adhesion molecule
- SEC-HPLC: Size Exclusion Chromatography-High pressure liquid chromatography

### Methods

### • Formulation of test article

The drug substance mAb17-1A was adsorbed to a 1% Al(OH)₃ suspension, the low dose formulation containing 5 µg, the lower dose formulation containing 0.5 µg and the lowest dose formulation containing 0.05 µg mAb17-1A per dose.

Components indicated in Table 1 were added under aseptic conditions and rotated end-over-end at 4°C in a 14 ml polypropylene-tube. After 15 min >99% of the drug substance had adsorbed onto Al(OH)₃ (measured by SEC-HPLC with Zorbax-GF250 from Agilent, data not shown) and the suspension was filled as 600 µl aliquots into 1 ml glass tubes, sealed and stored at 4°C.

**Table 1: Formulation of the vaccines**

| **Excipients** | **Quantity** | **Volume** | **Final** conc. |
|---|---|---|---|
| mAb17-1A, 0.1, 1 or 10 mg/ml | 12, 120 or 1200 µg | 12 µl | 0.1, 1 or 10 µg/ml |
| Thimerosal | 3.6 mg | 120.0 µl | 0.1 mg/ml |
| NaCl physiol. | | 9.858 µl | |
| Alhydrogel | 120.3 mg | 2.010 µl | 3.34 mg/ml |
| Σ | | 12,000 µl | |

### • Treatment of Rhesus monkeys

Immunization studies were performed at BioTest facilities (Conarovice, Czech Republic). Three groups of Rhesus monkeys (*Macacca mulatta*) each consisting of four individuals were vaccinated (*see* Table 2). The animals had body weights between 4 and 6 kg and had not been subjected to any pre-treatment. Monkeys were vaccinated four times by subcutaneous application of 500 µl on days 1, 15, 29 and 57 (appendix 1). One group of monkeys ("low dose vaccination group", animals N° 374, 377, 378 and 379) received 5 µg mAb17-1A per vaccination, the other ("lower dose vaccination group", animals N° 332, 334, 344 and 345) received 0.5 µg mAb17-1A at each vaccination and the third group ("lowest dose vaccination group", animals N° 335, 340, 346 and 350) received 0.05 µg mAb17-1A per vaccination.

Body weight of monkeys was recorded on day -3 and at the end of the study. Any signs of illness were recorded. Following every immunization, the application site of each monkey was checked for reddening, swelling or haematoma. In addition, each monkey was checked once a week for the colour and consistency of faeces, occurrence of sore, swollen or reddened eyelids. All parameters were documented (appendix 2).

**Table 2: Immunization group assignment**

| group | ID | medication | gender | Date of birth |
|---|---|---|---|---|
| P3/03 | 374 | 5 µg mAb17- 1A/dose | M | 19.02.200 1 |
| | 377 | | M | 16.02.200 1 |
| | 378 | | F | 21.02.200 1 |
| | 379 | | F | 19.03.200 1 |
| P4/03 | 332 | 0.5 µg mAb17- 1A/dose | M | 06.03.200 1 |
| | 334 | | M | 04.05.200 1 |
| | 344 | | F | 30.06.200 1 |
| | 345 | | F | 14.07.200 1 |
| P5/03 | 335 | 0.05µg mAb17- 1A/dose | M | 07.06.200 1 |
| | 340 | | M | 05.02.200 1 |
| | 346 | | F | 05.08.200 1 |
| | 350 | | F | 05.06.200 1 |

### • Blood sampling and handling of serum samples

Serum samples were collected on days 1 (preserum), 15, 29, 57 and 71.

Blood for serum preparation was collected into vials containing clotting activator, which were provided by Igeneon. The vials were left at room temperature for 30 minutes, and then centrifuged at 1500xg for 30 minutes and at least three aliquots transferred into 1.8ml vials.

Each aliquot of serum was labelled with the respective animal number, the study number, the study day and the date of bleeding. Serum samples were stored at -80°C at BioTest facilities until transported to Igeneon.

Serum samples were transported to Igeneon on dry ice and stored at -80°C for further use.

### • mAb17-1A ELISA

Pre- (sera until day 1) and immune sera (days 15, 29, 57, 71 or bleeding sera) were analyzed for the humoral immune response developed against mAb17-1A by mAb17-1A ELISA: mAb17-1A (10 µg/ml in coating buffer (PAA, Lot: T05121-436)) was coated onto microplates (Maxisorp® (NUNC) and blocked by incubation with 5% FCS (PAA, #A01223-384) in PBS def. Sera were applied in a dilution series (dilution factor 3, 6 steps starting at 1:60) in PBS supplemented with 2% FCS. Induced antibodies were detected by their constant domains using a goat-anti-human-IgG+A+M-HRP conjugate (Zymed, #62-8320) and stained with OPD (Sigma, #67H8941) in staining buffer (PAA, #T0521-437) using H₂O₂ (30%) as substrate according to the instructions by the manufacturer. Colour development was stopped by H₂SO₄ (30%). Extinction at 492 nm was measured using 620 nm as reference.

The titer is calculated against a titre of a positive control serum (animal from a previous immunization study, 8415 F), with an anti-mAb17-1A titer of 1:9000.

Seroconversion was defined as a titer of >1000.

### • Immuno affinity chromatography (IAC)

This analysis was performed to characterize and quantify the mAb17-1A specific immune response developed by the animals. Purification of immune sera was performed using immuno affinity chromatography (immunization antigen).

Affinity chromatography was performed according to standard procedures.

The system used was an AEKTA-explorer (Amersham Biosciences, Uppsala, Sweden). One ml of serum was diluted 1:10 with PBS def. 1x + 0.2 M NaCl, pH=7.2 ("buffer A"). After column equilibration the diluted serum was loaded onto the chromatography column with a flow rate of 1 ml/min. Unbound sample was washed out with buffer A until UV-line (280nm) was below 5 mAU. The elution of bound sample was performed by elution with 100 mM glycine-buffer pH=2.9 ("buffer B, elution buffer"). Fractions of interest were neutralized immediately with 1 M NaHCO₃ and analyzed by SEC-HPLC and preserved for further analysis by adding polyethylene glycol to a final concentration of 0.04%.

The affinity chromatography column used was *mAb17-1A Sepharose*^{®}*:* mAb17-1A coupled to a CH-Sepharose 4B (ligand density: 2mg/ml) column (XK10/2, Amersham Biosciences, Uppsala, Sweden). Immunoglobulins (IgG, IgM) were quantified by size exclusion chromatography (SEC-HPLC) on a ZORBAX GF-250 column (Agilent Technologies) on a DIONEX-system with 220 mM NaH₂PO₄ plus 10% CH₃CN as loading buffer. Effluent was monitored online at 214 nm.

A standard curve of human IgG and IgM (Pentaglobin^{®}) was used as a reference standard for quantification of immunoglobulins in eluted fractions.

### RESULTS

### • Overall titer of immune response

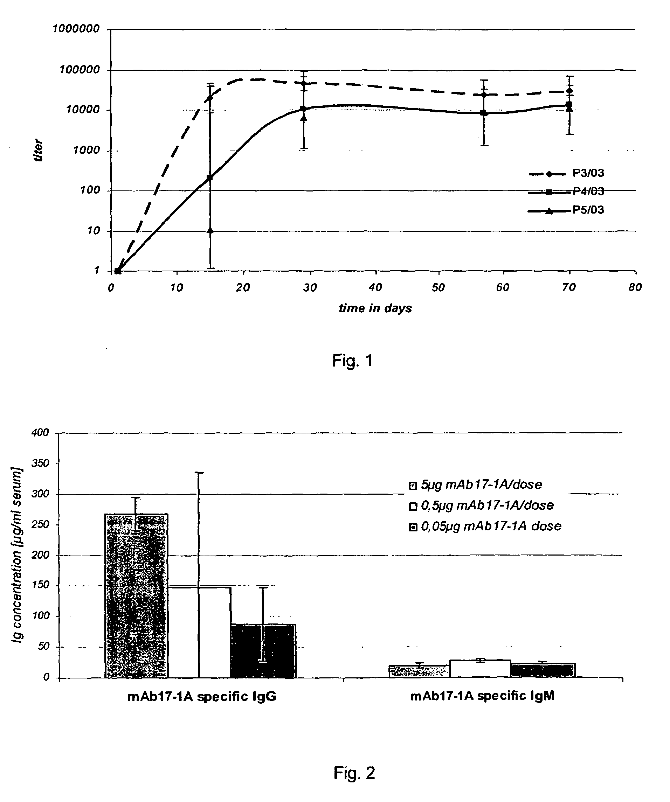

Pre-immune serum and immune sera of days 15, 29, 57 and 71 of the three dosage groups were analyzed for immune immunization antigen specific immune response. ELISA results are summarized in Table 1. Geometric mean values and scatter factors are indicated. A graphical display of geometric mean values and of the calculated tolerance intervals is shown in Figure 1.

**Table 1: Results of mAb17-1A ELISA of pre-immune and immune sera of all immunization groups, for geometric mean values calculation, below detection limit values (BDL) were replaced by the value 0.1.**

| ***animal*** | ***day 1*** | ***day 15*** | ***day 29*** | ***day 57*** | ***day 71*** |
|---|---|---|---|---|---|
| | ***Immunization group: 5* µ*g*/*dose*** | | | | |
| 374 | BDL | 29779* | 55440* | 25334 | 29529 |
| 377 | BDL | 37271* | 56970* | 21136* | 34951* |
| 378 | BDL | 13131* | 44323* | 20824* | 21426 |
| 379 | BDL | 10336* | 31303* | 32492 | 35381 |
| ***Geometric mean*** | ***0*** | ***19700*** | ***45753*** | ***24618*** | ***29741*** |
| Scatter factor | 1,0 | 1,9 | 1,3 | 1,2 | 1,3 |

| | ***Immunization group: 0.5* µ*g*/*dose*** | | | | |
|---|---|---|---|---|---|
| 332 | BDL | 1055 | 4290 | 4670 | 4578 |
| 334 | BDL | 5856* | 89952* | 49778* | 53450* |
| 344 | BDL | BDL | 2514 | 2117 | 5271 |
| 345 | BDL | 348* | 11787* | 11023* | 26201* |
| ***Geometric mean*** | ***0*** | ***215*** | ***10369*** | ***8582*** | ***13558*** |
| Scatter factor | 1,0 | 43,2 | 4,8 | 3,9 | 3,35 |

| | ***Immunization*** | ***group:*** | ***0.05*** | **µ*g*/*dose*** | |
|---|---|---|---|---|---|
| 335 | BDL | 12903* | 118283 * | 90269* | 64737* |
| 340 | BDL | BDL | 4654 | 6118 | 6407 |
| 346 | BDL | BDL | 495 | 1960* | 4815* |
| 350 | BDL | BDL | 6601 | 5690 | 8465 |
| ***Geometric mean*** | ***0*** | ***11*** | ***6512*** | ***8859*** | ***11403*** |
| Scatter factor | 1,0 | 113,6 | 9,5 | 5,1 | 3,25 |

| | | | | | |
|---|---|---|---|---|---|
| *serum was measured repeatedly, from these individual results mean value was taken | | | | | |

In the 5µg dose group titers against mAb17-1A became visible for all individuals on day 15 and increased to the highest titer by day 29 (two weeks after the second immunization). After the second vaccination (day 29) no significant titer increase was detectable, and the level of immune response was maintained by the third and fourth vaccination. According to the definition of seroconversion (titer >1000) 4 out of 4 monkeys of this treatment group had seroconverted by day 14. A quite similar titer endpoint (day 71) could be observed in the 0.5µg and the 0.05µg dose group. In the 0.5µg dose group, 2 out of four monkeys had seroconverted by day 15, the other two animals seroconverted by day 29. In the 0.05µg dose group only one animal had seroconverted on day 15, followed by two animals who seroconverted by day 29. All animals of the lowest dose group had seroconverted after the third vaccination (day 57). Compared to the kinetics shown by the 5µg dose group, induction of anti-mAb17-1A titers in the lower dose groups (0.5 and 0.05µg/dose) showed slightly slower kinetics with a peak value of the overall immune response at nearly the same level as the 5µg dose group. These differences are statistically not significant due to large confidence intervals caused by heterogeneity of group population.

Results are shown in Figure 1. The immunization antigen specificity was measured by mAb17-1A ELISA, immunization group specific geometric mean values are compared for all time point, 95% confidence intervals are indicated.

### • Characterization and quantification of immune response by affinity chromatography analysis

Immune sera (day 71) were affinity purified using mAb17-1A Sepharose^{®}. The amount of IgG and IgM was quantified by SEC-HPLC in comparison to standard curve of polyclonal immunoglobulins (Pentaglobin^{®}). Results are summarized in Table 2 and displayed graphically in Figure 2.

| | ***mAb17-1A specific IgG [µg*/*ml]*** | ***mAb17-1A spe- cific IgM [*µ*g*/*ml]*** |
|---|---|---|
| **Immunization group: 5 µg/dose** | | |
| 374 | 244 | 19 |
| 377 | 253 | 15 |
| 378 | 308 | 25 |
| 379 | 267 | 21 |
| *Average* | *268* | *20* |
| Stdev | 28 | 4 |

| | **Immunization group: 0,5 µg/dose** | |
|---|---|---|
| 332 | 75,5 | 32 |
| 334 | 467 | 30 |
| 344 | 66 | 26 |
| 345 | 204 | 25 |
| ***Average*** | ***203*** | ***28*** |
| Stdev | 187 | 3 |

| | **Immunization group: 0,05 µg/dose** | |
|---|---|---|
| 335 | 183 | 21 |
| 340 | 53 | 24 |
| 346 | 60 | 23 |
| 350 | 99 | 27 |
| ***Average*** | ***99*** | ***24*** |
| Stdev | 60 | 3 |

| | | |
|---|---|---|
| Table 2: Results of affinity purification of day 71 immune serum of immunization groups. Purified immunoglobulin is quantified by SEC-HPLC. | | |

Figure 2 shows the specificity of induced immune response analyzed by mAb17-1A affinity chromatography, IgG and IgM amounts were quantified by SEC-HPLC analysis, mean values of both groups and 95% confidence intervals are displayed.

All three formulations of mAb17-1A triggered a strong IgG type immunization antigen specific humoral immune response.

Analysis yielded an immunization antigen specific IgG amount of 268, 203 and 99 µg IgG µg/ ml serum for the 5, 0.5 and 0.05µg/ml immunization groups respectively. All individuals significantly increased anti-mAb17-1A titer seven- to 47-fold in comparison to a preformed IgG level of binding immunoglobulins in the pre-immune serum (about 10 µg/ml).

The mAb17-1A specific immune response of day 71 immune serum from the 5µg dose group was 30% higher than the one induced by the lower dose group (0.5 µg/dose) which was 100 % higher in comparison to the one induced by the 0.05µg dose. The mAb17-1A specific IgM level of pre- and immune serum in both treatment groups was nearly identical about 20-30 µg/ml. This level was not affected by the treatment.

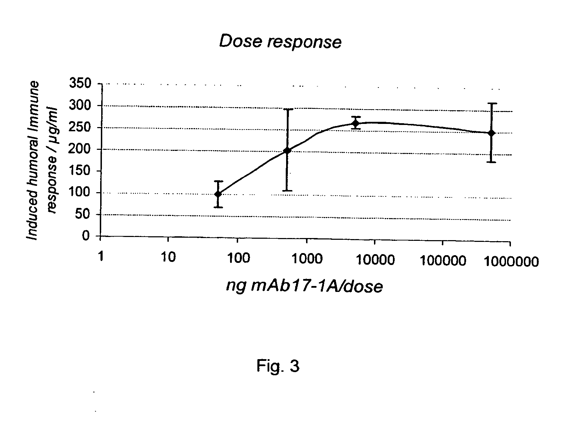

A graphical display of the dose dependent humoral immune response to mAb17-1A induced by vaccination (IgG titers measured by affinity chromatography 14 days after the last booster immunization) is shown in Figure 3. Data from previous studies (500 µg/dose) are included.

Figure 3 shows immunization antigen dose dependent induced humoral immune responses (IgG) measured by affinity chromatography 14 days after the last booster immunization, 95% confidence intervals are indicated

Amounts of induced immunization antigen specific IgG appeared to be dose dependent in a range from 0.05 to 5 µg/dose. Increased doses (until 500 µg/dose) did no more affect the IgG levels induced after three booster immunizations. Average IgG values induced by immunization antigen doses higher than 5 µg/dose did not exceed 250 µg/ml serum.

### Comparative dosage experiments using an anti-Lewis Y antibody

### Introduction

In this Rhesus monkey trial the immune responses after vaccination with an anti-idiotypic antibody (Ab2) (raised against an anti-LeY antibody (Ab1)) which mimics the Lewis Y carbohydrate antigen were analyzed regarding dependency of the vaccination effect on application dosis.

An immunogenic formulation of an anti-idiotypic antibody (Ab2) (raised against an anti-LeY antibody (Ab1)) which mimics the Lewis Y carbohydrate antigen was used for the experiments. The Lewis Y carbohydrate antigen is expressed on the surface of many human cancer cells of epithelial origin. Its limited expression in normal tissues makes LeY an attractive target for cancer immunotherapy. However, Lewis Y as a part of "self" is only poorly recognized by the immune system. Moreover, its carbohydrate nature is a further reason for the poor immunogenicity of the Lewis Y antigen.

The following immunization groups were vaccinated:

**Table 3**

| Formulation | Application Route | Applied Volume | Animal number | Animal Identifications |
|---|---|---|---|---|
| P11sc/02 | s.c. | 50µg/100µl | 3 | 25, 169, 262 |
| P12sc/02 | s.c. | 5µg/100µl | 3 | 112, 309, 157 |
| P12id/02 | i.d. | 5µg/100µl | 3 | 117, 143, 211 |

### Methods

### Vaccine

### Formulations of the anti-idiotypic antibody:

| | |
|---|---|
| Formulation [P12.../02] : | 5µg/100µl in1mM Na-phosphate, 0,86% NaCl, pH6.0 adsorbed on Alhydrogel; 0,15m1/vial; |
| Formulation [P11.../02] : | 50µg/100µl in1mM Na-phosphate, 0,86% NaCl, pH6.0 adsorbed on Alhydrogel; 0,15ml/vial; |

### Application

| | |
|---|---|
| Four applications: | on day 1, 15, 29 and 57 |
| Subcutaneous (s.c.): | [P12sc/02], [P11sc/02]: 100µl/animal |

### Animals

Rhesus monkeys (macacca mulatta); body weight between 4 and 6 kg; no pre-treatment of the animals; Animal number per group: 3 animals, with the exception:
Immunization scheme and bleeding
Bleedings on days -11, -7, -3, 1, 8, 15, 29, 57, 71 and 76. Bleeding was done before immunization.
Each bleeding should yield at least 4 ml sera (use SST Clotactivator-vials (Vacutainer)); serum has to be filled into Nunc vials 1,8ml (375418);
Purification of immune sera by affinity chromatography using the vaccine antigen as ligand (Anti-idiotypic antibody (Ab2) (raised against an anti-LeY antibody (Ab1)) which mimics the Lewis Y carbohydrate antigen).

Performed as described above.

The following affinity chromatography columns were used: Sepharose: Vaccine antigen used as ligand 3H4B5 coupled to a CH-Sepharose 4B column, Lot 170700. Hi Trap ProteinG-Column: ProteinG coupled to a column (Amersham Pharmacia, Cat. Nr. 17-0404-03)
Purification of Pre- or Immune sera was performed using single step affinity chromatography, and analysed using size exclusion chromatography (SEC).

After quantification of the amounts of immunoglobulins by SEC the remaining serum eluates were stabilized by addition of FCS (final conc.: 2%) and stored at +4°C.

Immune sera at selected time points (day 29, 57, 76) were analysed using affinity chromatography using the vaccine antigen as ligand followed by size exclusion chromatography (SEC).

The first table shows data for day 29: in all vaccinated groups Ig (IgG and IgM) were found in the immune serum. The immunization effect was clearly dose dependent with highest Ig (in particular IgG, highest IgG/Ig ratio) found in the high dose group (500µg) resembling the dose dependency found using the ELISA using the vaccine antigen as ligand.

**Table 4**

| Studies P10-12/02 | **Rhesus monkey #** | **IgM** **per ml Serum** | **IgG** **per ml Serum** | **Ig** **per ml Serum** | **IgG/Ig** **%** | **IgG/Ig** **%** |
|---|---|---|---|---|---|---|
| | | **[ug]** | **[ug]** | **[ug]** | | **mean** |
| P11 50µg sc | 262 | 18 | 65 | 83 | 78 | **62** |
| P11 50µg sc | 169 | 17 | 17 | 34 | 50 | |
| P11 50µg sc | 25 | 22 | 32 | 54 | 59 | |
| **Mean** | | **19** | **38** | **57** | | |
| SD | | 3 | 25 | 25 | | |
| P12 5µg sc | 157 | 19 | 25 | 44 | 57 | **56** |
| P12 5µg sc | 309 | 17 | 21 | 38 | 55 | |
| P12 5µg sc | 112 | 26 | 35 | 61 | 57 | |
| **Mean** | | **21** | **27** | **48** | | |
| SD | | 5 | 7 | 12 | | |
| P12 5µg id | 211 | 21 | 51 | 72 | 71 | **58** |
| P12 5µg id | 143 | 17 | 17 | 34 | 50 | |
| P12 5µg id | 117 | 12 | 13 | 25 | 52 | |
| **Mean** | | **17** | **27** | **44** | | |
| SD | | 5 | 21 | 25 | | |

Immune sera (day 57) were analysed using affinity chromatography using the vaccine antigen as ligand followed by size exclusion chromatography (SEC).

**Table 5**

| Studies P10-12/02 | **Rhesus monkey #** | **IgM** **per ml Serum** | **IgG** **per ml Serum** | **Ig** **per ml Serum** | **IgG/Ig** **%** | **IgG/Ig** **%** |
|---|---|---|---|---|---|---|
| | | **[ug]** | **[ug]** | **[ug]** | | **mean** |
| P11 50µg sc | 262 | 17 | 72 | 89 | 81 | **68** |
| P11 50µg sc | 169 | 28 | 43 | 72 | 60 | |
| P11 50µg sc | 25 | 20 | 34 | 54 | 63 | |
| **Mean** | | **22** | **50** | **72** | | |
| SD | | 6 | 20 | 18 | | |
| P12 5µg sc | 157 | 22 | 43 | 65 | 66 | **61** |
| P12 5µg sc | 309 | 16 | 27 | 43 | 63 | |
| P12 5µg sc | 112 | 27 | 30 | 57 | 53 | |
| **Mean** | | **22** | **33** | **55** | | |
| SD | | 6 | 9 | 11 | | |
| P12 5µg id | 211 | 16 | 27 | 43 | 63 | **55** |
| P12 5µg id | 143 | 20 | 17 | 38 | 45 | |
| P12 5µg id | 117 | 12 | 17 | 29 | 59 | |
| **Mean** | | **16** | **20** | **37** | | |
| SD | | 4 | 6 | 7 | | |

Immune sera (day 76) were analysed using affinity chromatography using the vaccine antigen as ligand followed by size exclusion chromatography (SEC). The data for day 57 and day 76 were very similar to those obtained at day 29 after immunization. In all vaccinated groups Ig (IgG and IgM) were found in the immune sera. The immunization effect was clearly dose dependent with highest Ig (in particular IgG, highest IgG/Ig ratio) found in the high dose group (500µg, data not shown).

**Table 6**

| Studies P10-12/02 | **Rhesus monkey** | **IgM** **pro ml Serum** | **IgG** **pro ml Serum** | **Ig** **pro ml Serum** | **IgG/Ig** **% g** | **IgG/I %** |
|---|---|---|---|---|---|---|
| | | **[µg]** | **[µg]** | **[µg]** | | **mean** |
| P11 50µg sc | 262 | 13 | 43 | 56 | 77 | **71** |
| P11 50µg sc | 169 | 21 | 46 | 67 | 69 | |
| P11 50µg sc | 25 | 15 | 30 | 45 | 67 | |
| **Mean** | | **16** | **40** | **56** | | |
| SD | | 4 | 9 | 11 | | |
| P12 5µg sc | 157 | 14 | 25 | 39 | 64 | **68** |
| P12 5µg sc | 309 | 15 | 55 | 70 | 79 | |
| P12 5µg sc | 112 | 18 | 30 | 48 | 63 | |
| **Mean** | | **16** | **37** | **52** | | |
| SD | | 2 | 16 | 16 | | |
| P12 5µg id | 211 | 15 | 43 | 58 | 74 | **65** |
| P12 5µg id | 143 | 19 | 19 | 38 | 50 | |
| P12 5µg id | 117 | 10 | 26 | 36 | 72 | |
| **Mean** | | **15** | **29** | **44** | | |
| SD | | 5 | 12 | 12 | | |

In comparison selected presera (day-11) were analyzed using affinity chromatography using the vaccine antigen as ligand followed by size exclusion chromatography (SEC).

As has to be expected significantly lower amounts of immunoglobulin (IgM, IgG) were found in the preserum in comparison to the according immune serum (Table 7).

**Table 7**

| Studies P10-12/02 | **Affe** | **IgM** **pro ml Serum** | **IgG** **pro ml Serum** | **Ig** **pro ml Serum** | **IgG/Ig** **%** | **IgG/Ig** **%** |
|---|---|---|---|---|---|---|
| | | **[µg]** | **[µg]** | **[µg]** | | **mean** |
| P12 5µg sc | 309 | 11 | 9 | 20 | **45** | |
| P12 5µg id | 143 | 10 | 6 | 16 | **38** | |

## Claims

1. Use of an antibody directed against the cellular membrane antigen EpCAM for the production of a pharmaceutical preparation for the prophylactic and/or therapeutic active immunization against cancer, **characterized in that** the antibody is in an amount of 1 ng/dose to 9 µg/dose.

2. Use according to claim 1, **characterised in that** the antibody concentration is in the range of 0.01 µg/dose to 9 µg/dose.

3. Vaccine containing an antibody against the cellular membrane antigen EpCAM for use the vaccination against cancer, **characterized in that** the antibody is in an amount of 1 ng to 9 µg.

4. Vaccine according to claim 3, **characterised in that** the antibody concentration is in the range of 0.01 µg to 9 µg.

5. Use or vaccine of any one of claims 1 to 4, wherein the antibody is of animal origin.

6. Use or vaccine of any one of claim 1 to 5, wherein the antibody is a monoclonal antibody.

7. Use or vaccine of any of claims 1 to 6, wherein the antibody is the murine monoclonal antibody mAb17-1A.

8. Use or vaccine of any one of claims 1 to 7, wherein 2 or more antibodies which are directed against different cellular membrane antigens or against different epitopes of a membrane antigen are used in combination with each other.

9. Use or vaccine of any one of claims 1 to 8, wherein the pharmaceutical composition further comprises also at least one vaccine adjuvant.

10. Use or vaccine of claim 9 wherein the vaccine adjuvant is aluminium hydroxide.

11. Use or vaccine of any one of claims 1 to 10, wherein the pharmaceutical composition is suitable for the administration by subcutaneous, intradermal or intramuscular injection.

12. Use or vaccine of an antibody according to any of claims 1 to 11, wherein antigenic structures can be coupled to the antibody.

13. Use or vaccine of an antibody according to claim 12, wherein the antigenic structures are tumor associated antigens.

14. Use or vaccine of an antibody according to claim 13, wherein the antigenic structures are carbohydrates selected from the group consisting of SialylTn, Lewis Y, Globo H.

## Patentansprüche

1. Verwendung eines Antikörpers, der gegen das zelluläre Membranantigen EpCAM gerichtet ist, zur Herstellung eines pharmazeutischen Präparats zur prophylaktischen und/oder therapeutischen aktiven Immunisierung gegen Krebs, **dadurch gekennzeichnet, dass** der Antikörper in einer Menge von 1 ng/Gabe bis 9 µg/Gabe vorhanden ist.

2. Verwendung gemäß Anspruch 1, **dadurch gekennzeichnet, dass** die Antikörper-Konzentration im Bereich von 0,01 µg/Gabe bis 9 µg/Gabe liegt.

3. Vakzin, welches einen Antikörper gegen das zelluläre Membranantigen EpCAM enthält, zur Verwendung bei der Impfung gegen Krebs, **dadurch gekennzeichnet, dass** der Antikörper in einer Menge von 1 ng bis 9 µg vorhanden ist.

4. Vakzin gemäß Anspruch 3, **dadurch gekennzeichnet, dass** die Antikörper-Konzentration im Bereich von 0,01 µg bis 9 µg liegt.

5. Verwendung oder Vakzin gemäß einem der Ansprüche 1 bis 4, wobei der Antikörper animalischen Ursprungs ist.

6. Verwendung oder Vakzin gemäß einem der Ansprüche 1 bis 5, wobei der Antikörper ein monoklonaler Antikörper ist.

7. Verwendung oder Vakzin gemäß einem der Ansprüche 1 bis 6, wobei der Antikörper der mausartige monoklonale Antikörper mAb17-1A ist.

8. Verwendung oder Vakzin gemäß einem der Ansprüche 1 bis 7, wobei 2 oder mehr Antikörper, die gegen unterschiedliche zelluläre Membranantigene oder gegen unterschiedliche Epitope eines Membranantigens gerichtet sind, in Kombination miteinander verwendet werden.

9. Verwendung oder Vakzin gemäß einem der Ansprüche 1 bis 8, wobei die pharmazeutische Zusammensetzung des Weiteren ebenfalls zumindest ein Vakzin-Adjuvans umfasst.

10. Verwendung oder Vakzin gemäß Anspruch 9, wobei das Vakzin-Adjuvans Aluminiumhydroxid ist.

11. Verwendung oder Vakzin gemäß einem der Ansprüche 1 bis 10, wobei die pharmazeutische Zusammensetzung für die Verabreichung durch subkutane, intradermale oder intramuskuläre Injektion geeignet ist.

12. Verwendung oder Vakzin eines Antikörpers gemäß einem der Ansprüche 1 bis 11, wobei antigene Strukturen an den Antikörper gebunden werden können.

13. Verwendung oder Vakzin eines Antikörpers gemäß Anspruch 12, wobei die antigenen Strukturen tumorassoziierte Antigene sind.

14. Verwendung oder Vakzin eines Antikörpers gemäß Anspruch 13, wobei die antigenen Strukturen Kohlenhydrate, ausgewählt aus der Gruppe bestehend aus SialylTn, Lewis Y, Globo H., sind.

## Revendications

1. Utilisation d'un anticorps dirigé contre l'antigène de membrane cellulaire EpCAM pour la production d'une préparation pharmaceutique pour l'immunisation active prophylactique et/ou thérapeutique contre le cancer, **caractérisée en ce que** l'anticorps est présent en une quantité de 1 ng/dose à 9 µg/dose.

2. Utilisation suivant la revendication 1, **caractérisée en ce que** la concentration en anticorps est comprise dans l'intervalle de 0,01 µg/dose à 9 µg/dose.

3. Vaccin contenant un anticorps contre l'antigène de membrane cellulaire EpCAM, destiné à être utilisé dans la vaccination contre le cancer, **caractérisé en ce que** l'anticorps représente une quantité de 1 ng à 9 µg.

4. Vaccin suivant la revendication 3, **caractérisé en ce que** la concentration en anticorps est comprise dans l'intervalle de 0,01 µg à 9 µg.

5. Utilisation ou vaccin suivant l'une quelconque des revendications 1 à 4, dans lequel l'anticorps est d'origine animale.

6. Utilisation ou vaccin suivant l'une quelconque des revendications 1 à 5, dans lequel l'anticorps est un anticorps monoclonal.

7. Utilisation ou vaccin suivant l'une quelconque des revendications 1 à 6, dans lequel l'anticorps est l'anticorps monoclonal murin mAb17-1A.

8. Utilisation ou vaccin suivant l'une quelconque des revendications 1 à 7, dans lequel deux ou plus de deux anticorps qui sont dirigés contre des antigènes de membrane cellulaire différents ou contre des épitopes différents d'un antigène membranaire sont utilisés en association les uns avec les autres.

9. Utilisation ou vaccin suivant l'une quelconque des revendications 1 à 8, dans lequel la composition pharmaceutique comprend en outre également au moins un adjuvant pour vaccin.

10. Utilisation ou vaccin suivant la revendication 9, dans lequel l'adjuvant pour vaccin est l'hydroxyde d'aluminium.

11. Utilisation ou vaccin suivant l'une quelconque des revendications 1 à 10, dans lequel la composition pharmaceutique est apte à l'administration par injection sous-cutanée intradermique ou intramusculaire.

12. Utilisation ou vaccin suivant l'une quelconque des revendications 1 à 11, dans lequel des structures antigéniques peuvent être couplées à l'anticorps.

13. Utilisation ou vaccin suivant la revendication 12, dans lequel les structures antigéniques sont des antigènes associés aux tumeurs.

14. Utilisation ou vaccin suivant la revendication 13, dans lequel les structures antigéniques sont des glucides choisis dans le groupe consistant en SialylTn, Lewis Y, Globo H.
